# EUROPEAN PATENT APPLICATION

(11) **EP 1 779 898 A1**
(43) Date of publication of application: **02.05.2007**
(21) Application number: 06255511.5
(22) Date of filing: 26.10.2006
(51) Int. Cl.: A61Q 9/00, A61K 8/18, A61K 8/25, A61K 8/26, A61K 8/19, A61K 8/73, A61K 8/89

(54) **Method of epilation**

(30) Priority: 27.10.2005 US 260697
(71) Applicant: McNeil-PPC, Inc., Skillman, NJ 08558-9418 (US)
(72) Inventor: Taneri, Jim, Flemington, NJ 08822 (US); Chang, Yi Hsin, Costa Mesa, CA 92626 (US); Akyuz, Rafael, Palos Verdes Estates, CA 90274 (US); Goldsberry, Susan, Huntington Beach, CA 92648 (US)
(74) Representative: Fisher, Adrian John

(57) **Abstract**

A method of preparing a body surface for epilation is provided. The method includes, prior to said epilation, applying to the body surface a composition that is adapted to leave a powder residue on the body surface. The composition includes a fluid vehicle Compositions of the present invention may include an insoluble solid, a fluid vehicle, and an analgesic.

## Description

### FIELD OF THE INVENTION

This invention relates to methods and compositions useful for epilation, in particular, to methods and compositions useful for preparing a body surface for epilation.

### BACKGROUND OF THE INVENTION

For aesthetic or personal reasons, many people find it desirable to remove unwanted hair from various areas of the human body, e.g., the bikini area, or areas of the face such as around the eyebrows or lips. Methods known for removing hair include shaving, chemical treatment, and physical hair removal. Shaving the hair suffers from the inconvenience of frequently needing to repeat the process, e.g., daily. The use of chemical treatments to cleave the hair shaft (i.e., depilatories) suffers from the drawback of using the chemicals themselves, which generally irritate the skin, have unpleasant odors, and are messy. Physical hair removal, in which hair is pulled from the skin "by the roots" (broadly termed "epilation") generally overcomes the problems of chemical irritation, smell, and the requirement for frequent treatment, but other additional problems are introduced. In particular, epilation can be painful to the subject, may result in not all of the treated hairs to be removed, and may be inconvenient to remove the epilation materials from the skin.

Since epilation can be traumatic, painful, and irritating and often requires additional time to remove the epilation materials, it is desirable to have a composition that can be topically applied to the skin that addresses one or more of these drawbacks. It has surprisingly been discovered that, prior to epilation, by applying a composition that includes a fluid vehicle and is adapted to leave a powder residue on the skin and hair, one or more of hair removal, pain mitigation, and convenience of cleanup are improved. Further, without wishing to be bound by theory, it is believed that the powder residue left behind on the skin and hair provides an electrostatic charge to the hair which causes the hair to stand up, improving hair removal.

### SUMMARY OF INVENTION

Accordingly, the invention relates to a method of preparing a body surface for epilation, said method including prior to said epilation, applying to said body surface a composition that is adapted to leave a powder residue on said body surface, wherein said composition comprises a fluid vehicle.

In another aspect of the invention, an oil in water emulsion composition including an insoluble solid, a fluid vehicle, and, an analgesic is provided.

### DETAILED DESCRIPTION OF THE INVENTION

The invention relates to a method of preparing a body surface for epilation, the method including topically applying a composition to the body surface prior to epilation. The composition includes a fluid vehicle and is adapted to leave a powder residue on the body surface.

By "epilation" it is meant the physical and typically forceful removal of the entire hair shaft hair from the body. Epilation includes what is commonly referred to as "waxing;" e.g., applying materials that bond to the hair, then applying sufficient force to extract the hair from the body. Epilation materials may include, for example waxes, polymers, resins, rosin sugar based products or other bonding materials, optionally compounded with oils, fillers, dyes, and the like. The epilation materials may be heated, even melted to provide tack and fluidity to improve contact and boding with the hair to be removed. The term epilation is also meant to encompass the use of mechanical devices to grasp the hairs and extract them from the body.

According to embodiments of the present invention, a body surface is prepared for epilation. By "body surface" it is meant that portion of the body encompassing a surface of the body from which unwanted hairs protrude (i.e., skin) and/or the hairs protruding therefrom. Examples of body surfaces include the bikini area, legs, arms and/or areas of the face such as around the eyebrows or lips.

Compositions of the present invention are adapted to leave a powder residue on the body surface to be treated. By "powder residue", it is meant a divided or particulate solid material that is at least partially derived from the composition, which is capable of resting on or sticking to the body surface. The powder residue may be formed from particles or agglomerates of particles that have a size, e.g., a mean or median diameter, of less than about 500 microns, more preferably less than about 100 microns, most preferably less than about 50 microns, such as from about 1 micron to about 50 microns. The particles may be of varying shapes, such as spherical, rod-like, lamellar, dendritic, disc-like, cylindrical, and the like. The particles may be associated with, such as loosely bonded to a thin film of other components present in the composition and/or sebum, that may be present on the skin or hair.

By "adapted to leave a powder residue" it is meant that when the composition is topically applied to the body surface a powder residue is generated. For example, the composition can be applied by gently rubbing the composition into the body surface that anchors the hairs to be removed, and waiting a period of time sufficient to permit the powder residue to form, for example, about 30-60 seconds. A typical "dose" in grams per square inch of skin is about 17 mg/square inch.

In order to ensure that the composition is adapted to leave a residue, the composition includes a powder residue-generating material. Suitable powder residue-generating materials include water-insoluble solids, water-soluble salts, and combinations thereof. In certain preferred embodiments, the powder residue-generating material includes a water-insoluble solids and, optionally, water-soluble salts. In certain particularly preferred embodiments, the powder residue-generating material is present in the composition in a weight percentage relative to the total composition, of at least about 1%, such as from about 1% to about 98%, preferably from about 3% to about 60%, more preferably from about 5% to about 40%, most preferably from about 15% to about 35%. Note that all percentages in this specification are percentages by weight, unless otherwise stated.

By "water-insoluble solid," it is meant a solid material that has a solubility of less than about 1% by weight in deionized water at 20° C. One example of a suitable water-insoluble solid includes inorganic particulates, such as inorganic oxides, nitrides, or silicates. Examples include such as silicon oxides, aluminum oxides, zinc oxides, titanium oxides, boron nitrides, talc, gypsum, calcite among others, and combinations thereof. It is desirable that the inorganic particle not be overly abrasive to the skin. The inorganic particle may be coated with a hydrophobic material. Particularly preferred examples of inorganic particles include silicon oxides, such as silica microspheres commercially available as SILICA from Kobo Products, Inc. of South Plainfield, NJ and boron nitride treated with polydimethylsiloxane, commercially available from Advanced Ceramics Corporation of Cleveland, OH as SOFTTOUCH boron nitride grade CC 6064. In certain particularly preferred embodiments, the inorganic particulate is present in the composition from about 0% to about 98%, preferably from about 0.5% to about 50%, more preferably from about 0.5% to about 20%, most preferably from about 1% to about 8%.

Another example of a suitable water-insoluble solid is an organic particualte. By "organic particulate" it is meant a predominantly organic material that meets the above requirements for powder residue. Suitable examples include synthetic organic materials or those of natural origin. Suitable organic particulates of natural origin include proteinacious particulates such as those derived from or including protein from various plants such as soy, wheat, oats, or other grains or particulates including water-insoluble carbohydrates, including cellulosic materials. Suitable cellulosic materials include those derived from exoskeletons such as chitin or vegetable derived cellulosics such as starches, including those derived from corn, potato, tapioca, and the like. Starches such as aluminum starch octenylsuccinate, e.g., DRY FLO PC, and tapioca starch, commercially available as TAPIOCA PURE are particularly notable. Both DRY FLO PC and TAPIOCA PURE are commercially available from National Starch and Chemical of Bridgewater, NJ. As discussed above, it is believed that the powder residue left behind on the skin and hair provides an electrostatic charge to the hair which causes the hair to stand up, improving hair removal. Without wishing to be bound by theory it is believed that corn starches and chemically modified corn starches are more likely to have a higher electrostatic charge as compared to tapioca starches. Accordingly, in one embodiment, the organic particulate is derived from corn starch. In certain particularly preferred embodiments, the organic particulate is present in the composition from about 0% to about 98%, preferably from about 1 % to about 60%, more preferably from about 2% to about 50%, most preferably from about 5% to about 25%.

In one embodiment of the invention the water insoluble solid includes a silicone particulate. By "silicone particulate," it is meant a material that is solid at room temperature and includes both siloxane bonds as well as silicon bonded to a carbon or carbon chain. Suitable silicone particulates include particulates having a core that includes silicone polymers and copolymers, such as powders formed from silicone elastomers, silicone resins, and combinations thereof. One particularly suitable solid silicone includes a silicone elastomer coated with a silicone resin: a vinyl dimethicone/methicone silsesquioxane crosspolymer, commercially available as KSP-100, from Shin-Etsu Chemical, of Tokyo, Japan. In certain particularly preferred embodiments, the silicone particulate is present in the composition from about 0% to about 98%, preferably from about 0% to about 25%, more preferably from about 0.5% to about 10%, most preferably from about 1% to about 3%.

In one embodiment of the invention the powder residue generating material includes a water-soluble salt. By "water-soluble salt," it is meant a material that is (1) capable of ionizing when placed in deionized water at 20° C and dissolving into said water at a concentration of greater than about 1% by weight and (2) capable of forming a divided solid or crystal upon evaporation of said water. Suitable water-soluble salts include salts of alkali metals (e.g., water soluble salts of sodium and potassium), salts of alkaline earth metals (e.g., water soluble salts of calcium and magnesium), water-soluble ionic polymers (e.g., polyacrylates such as CARPOBOL, or other polymers having ionic groups such as sulfates, sulfonates, carboxylates, borates and the like), and water-soluble ionic surfactants. In certain particularly preferred embodiments, the water-soluble salt is present in the composition from about 0% to about 98%, preferably from about 1% to about 50%, more preferably from about 1% to about 10%, most preferably from about 2% to about 6% Ionic surfactants are particularly notable water-soluble salts that may be useful in compositions of the present invention. These materials are useful not only in increasing the powder residue in the formula, but also are advantageous in stabilizing the formulation, e.g. in dispersing the powders used in the formula. Suitable water soluble ionic surfactants include those anionic, cationic, and amphoteric surfactants (amphoteric surfactants, when used in the composition are capable of forming one or more of cationic, anionic, and zwitterionic moieties) that are commonly used in personal care formulations in "leave-on" products to (1) co-emulsify water and oil phases (2) provide stabilized dispersion of powders and particles, and/or (3) reduce the surface tension of aqueous systems. Since cationic surfactants may form unstable complexes in the composition, anionic and amphoteric surfactants are the preferred ionic surfactants. Anionic surfactants are particularly notable, and, while numerous anionic surfactants may be suitable, fatty acid esters, sulfosuccinate esters, isethionates, esters of phosphoric acid, carboxylic acid salts are particularly notable. One example of a suitable phosphate ester is dicetyl phosphate, ceteth-10 phosphate, sold as a mixture with cetearyl alcohol as CRODAPHOS CES, commercially available from Croda, Inc of Edison, NJ. One example of a suitable fatty acid ester is propylene glycol isoceteth-3-acetate, commercially available as HETESTER PHA from Bernel Chemical Co., Division of ALZO International Inc., Sayreville, NJ.

In certain particularly preferred embodiments, the ionic surfactants are present in the composition from about 0% to about 15%, preferably from about 0% to about 5%, more preferably from about 0% to about 4%, most preferably from about 1% to about 4%. Care should be taken, if water-soluble salts are included in the composition, particularly water-soluble polymers, that these salts do not de-stabilize the composition.

The powder-generating material has been discussed above, with particular examples of water-insoluble solids and water-soluble salts provided. The composition may additionally include non-powder generating materials, e.g., materials that are generally non-volatile and primarily non-ionic and/or film-forming (as opposed to powder generating materials, which are generally ionic and/or particulate). However, in one notable embodiment of the invention the amount of non-powder generating materials in the composition is limited.

In one embodiment of the invention, the composition includes a "weight solids fraction" of powder generating material that is at least about 15% of a total solids concentration in the composition. In a preferred embodiment, the weight solids fraction of powder generating materials in the composition is from about 30% to about 99% more preferably from about 50% to about 98%, and most preferably from about 65% to about 85%.

"Weight solids fraction" of powder generating materials may be determined by first measuring or calculating the "percent solids," i.e., the weight percentage of material in the composition that is non-volatile as determined by using conventional methods including, for example, a moisture analyzer such as a Mettler Toledo, Halogen Moisture Analyzer HB 43 available from Mettler-Toledo, Inc., Colombus, OH wherein a two gram sample of composition is placed in the machine at 120 °C. The machine stops when the weight becomes constant usually taking approximately 15-20 minutes. The weight solids fraction of the powder generating materials is the weight percentage of the powder generating materials in the composition divided by the percent solids and is therefore an indicator of how much of the composition includes powder generating materials.

The percent solids of the composition may be from about 1 % by weight to about 98% by weight, preferably from about 3% to about 60%, more preferably from about 5% to about 50%, and most preferably from about 10% to about 35%.

Without wishing to be bound by theory, it is believed that non-powder generating materials, especially, volatile materials, may have behavior on the body surface (e.g., stickiness, tack, etc.) that may reduce adhesion of the powder and/or adhesion or stickiness of the epilation materials, thereby reducing the effectiveness of epilation. Accordingly, the amount of volatile materials present in the compositions must be carefully balanced so as not to avoid interference with stickiness of the epilation material. Non-powder generating materials include generally non-volatile and primarily non-ionic and/or film-forming materials. Non-powder material may be liquid or solids at room temperature. Non-limiting examples of non-powder-generating materials include mineral oils, petrolatum, vegetable oils (glyceryl esters of fatty acids, triglycerides), fatty alcohols, waxes and other mixtures of esters that are not necessarily esters of glycerol; polyethylene and non-hydrocarbon based oils such as liquid silicones such as dimethicone (polydimethylsiloxane), silicone oils; non-ionic surfactants, and various non-ionic water-soluble polymers that are used as thickeners or film-formers.

Due to the particular slipperiness of certain ingredients such as liquid silicones, in certain preferred embodiments the composition is substantially free of these ingredients. By "substantially free of liquid silicones," it is meant that the compositions include less than about 3%, preferably less than about 2%.It is believed that by limiting the level of liquid silicones, the ability of the epilation wax to grip the hairs is enhanced and hair removal is improved.

The inventors have noted that in order to provide a powder residue and to achieve spreadability of the composition across the hair and skin, the composition should include a fluid vehicle. By "fluid vehicle" it is meant one or more moieties that are liquid at room temperature and capable of spreading the powder-generating materials across the body surface. The fluid vehicle preferably includes water. The fluid vehicle, in addition to or even in place of water, may include other moieties that are liquids at 20° C., particularly those that have one or more functionalities such as emolliency, humectancy, emulsification, dispersion, microbial preservation, fragrance, or other functions as discussed as discussed below. The fluid vehicle may be present in the composition in a concentration by weight from about 10% to about 99%. However, to achieve improved ability to spread the powder across the body surface the fluid vehicle is preferably present in the composition in a concentration by weight from about 40% to about 98%, more preferably from about 55% to about 90%, most preferably from about 50% to about 80%.

In order to enhance the phase stability of the composition, the inventors have found that a freezing point depressant may be included, particularly when a water-insoluble solid is present. Suitable freezing point depressants include polyhydric alcohols, such as but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is a particularly notable freezing point depressant. When included the freezing point depressant may be present in a concentration by weight from about 0.5% to about 5%, more preferably from about 1% to about 3%.

The composition may include emollients, i.e., ingredients that either remain on the body surface or penetrate therethrough, acting as lubricants, and to improve the feel on the skin; and/or and humectants for improving water retention of the skin. Suitable emollients include fatty esters, fatty alcohols, mineral oil, and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof. Suitable humectants include polyhydric alcohols, examples of which are previously noted.

In order to promote phase stability of the composition a stabilizing agent, such as a rheology modifier or thickener may be included. The rheology modifier may be, for example a clay or polymer. The inventors have noted that in order to enhance phase stability the composition may include a non-ionic polymer, such as a naturally occurring polysaccharides such as xanthan gum (e.g. KELTROL CG), alginates, carageenan; cellulose ethers including methyl cellulose, carboxymethyl cellulose, hydroxy propyl methyl cellulose, hydroxyethyl cellulose, hydroxypropyl cellulose, and ethyl hydroxyethyl cellulose, guar gum, among others. Xanthan gum is particularly notable. In one embodiment, the concentration of non-ionic polymer is less than about 1% by weight.

The inventors have noted that it is particularly desirable to have one or more classes of functional ingredients such as analgesics, anti-inflammatory agents, ant-edema agents, conditioning agents, such as, hydrolyzed soy protein, commercially available as HYDROSOY 2000 from Croda, Inc of Edison, NJ. and counterirritants within the composition. For example, in order to reduce pain associated with epilation, the composition may further include an analgesic or topically active, pain-mitigating compound. Suitable analgesics include, for example, "caine" molecules such as benzocaine, dibucaine, lidocaine; benzyl alcohol, camphor, juniper tar, menthol, methyl nicotinate, methyl salicylate, phenol, resorcinol, and mixtures thereof. In certain preferred embodiments, the composition includes an analgesic in a concentration from about 0.5% to about 5%, more preferably from about 1% to about 2%. In one notable embodiment, the analgesic is a "caine" molecule, most preferably lidocaine. In certain preferred embodiments, the lidocaine is present as lidocaine base, as such, in these embodiments, the composition has a pH from about 7.0 to about 9.0, such as from about 7.0 to about 8.0.

In order to reduce inflammation associated with epilation, the composition may further include an anti-inflammatory agent. Examples of suitable anti-inflammatory agents nonexclusively include benoxaprofen, centella asiatica, bisabolol, extracts of feverfew, green tea extract, green tea concentrate, hydrogen peroxide, salicylates, oat oil, chamomile, dipotassium glycyrrhizate, and mixtures thereof. The anti-inflammatory agent may be present in the composition in a concentration from about 0.05% to about 2%, such as from about 0.1 % to about 1%.

In order to reduce edema associated with epilation, the composition may further include an anti-edema agent. Examples of suitable anti-edema agents nonexclusively include bisabolol natural, synthetic bisabolol, corticosteroids, beta-glucans, and mixtures thereof. The anti-edema agent may be present in the composition in a concentration from about 0.05% to about 2%, such as from about 0.1% to about 1%.

In order to reduce irritation associated with epilation, the composition may further include a counterirritant. Examples of suitable counterirritants nonexclusively include allantoin, camphor, menthol, methyl salicylate, peppermint and clove oils, ichtammol, and mixtures thereof. The counterirritant may be present in the composition in a concentration from about 0.05% to about 2%, such as from about 0.1% to about 1%.

The compositions according to the invention can include additional ingredients commonly found in skin care compositions, such as for example, preservatives, antioxidants such as vitamin E, fragrances, sequestering agents such as EDTA and the like, pH adjusters/buffers, etc., each in concentrations that are typical for personal care compositions, provided that the ingredients are physically and chemically compatible with the other components of the composition.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In one embodiment, the preservative is mixture of methylparaben, propylparaben, and phenoxyethanol.

Compositions of the present invention may be substantially free of chemical hair removers (e.g., ingredients that are designed to chemically cleave or dissolve the hair shaft) and/or free of bleaches. Chemical hair removers include, for example, thioglycolic acid and its salts. Bleaches include, for example, peroxygen chemicals. By "substantially free" of these ingredients, it is meant that the compositions include less than about 0.5%. In one embodiment of the invention, these ingredients are completely absent from the composition.

Compositions of the present invention may have a pH and a viscosity that is variable. In certain preferred embodiment, the composition has a pH from about 6 to about 9, such as from about 6.5 to about 8.0.

The inventors have noted that for enhanced aesthetics and ability to spread the composition across the body surface, the fluid vehicle may include a water-phase and an oil phase. In one embodiment of the invention, the fluid vehicle includes an oil phase emulsified in the water phase, and may be, for example, a O/W or a W/O/W emulsion. In such an embodiment, the oil phase can include various hydrophobic liquids as well as other materials soluble therein, including mineral oils, petrolatum, vegetable oils (glyceryl esters of fatty acids, triglycerides), fatty alcohols, waxes and other mixtures of esters that are not necessarily esters of glycerol; polyethylene and non-hydrocarbon based oils such as liquid silicones such as dimethicone (polydimethylsiloxane), silicone oils. In certain preferred embodiment the composition is an O/W emulsion, which provides good aesthetics/low greasiness. The inventors have unexpectedly found that the composition can be formulated as a stable O/W emulsion with a relatively high proportion of water-insoluble solid dispersed therein, such as may be characterized as having a weight solids fraction of at least about 15% of a powder generating material, more preferably wherein the weight solids fraction is from about 65% to about 95%. The inventors have further unexpectedly noted that the composition may be formulated in a phase stable manner including beneficial ingredients such as, for example, lidocaine.

Compositions of the present invention may be made by techniques known in the art by blending, dispersing, emulsifying, and/or mixing the various ingredients. The above described composition may be prepared by combining the desired components in a suitable container and mixing them under ambient conditions in any conventional mixing means well known in the art, such as a mechanically stirred propeller, paddle, and the like.

Generally, the composition is topically applied to a body surface (skin and/or hairs) that includes unwanted hair such as the bikini area, or areas of the face such as around the eyebrows or lips. Application may be via the hand or via an applicator such as a brush, wand, sponge, fibrous pad, and the like. The composition is rubbed in sufficiently and a time period is allowed to elapse such as to allow the body surface to dry. An epilation treatment is then performed, such as by applying an epilation wax and forcefully pulling and extracting the hair from the body surface.It has been discovered that when a body surface is prepared for epilation according to the present invention, the epilation process is less painful, results in an increase in hair removal, and less of the material that is bounded to the hair, e.g., wax, is left behind on the body surface. Accordingly, in another embodiment, the invention relates to a process for mitigating the pain, trauma and/or irritation associated with epilation, a process for improving hair removal of an epilation process and a process for decreasing the amount of wax left behind on the body surface after epilation.

Further, without wishing to be bound by theory it is believed that the that the powder residue left behind on the skin and hair provides an electrostatic charge to the hair which causes the hair to stand up, improving hair removal. Accordingly, in another embodiment the invention relates to a method for providing an electrostatic charge to a body surface, said method comprising applying to said body surface a composition that is adapted to leave a powder residue on said body surface, wherein said composition comprises a powder residue generating material and a fluid vehicle.

In yet another embodiment, the invention relates to a method for ameliorating redness or inflammation of mammalian skin by topically applying a composition that is adapted to leave a powder residue on said body surface, wherein said composition comprises an insoluble solid, a fluid vehicle, and, an analgesic.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

### EXAMPLE 1

The following formula was made in accordance with the teachings of this invention.

| **ingredient Trade Name*** | **CTFA/INCl Name** | **Primary Function** | **%** | **% Active** | **% Solids** |
|---|---|---|---|---|---|
| **PART A** | | | | | |
| Deionized Water | Water (Aqua) | Solvent | 58.1900 | 0 | 0 |

| **PART B** | | | | | |
|---|---|---|---|---|---|
| Keltrol CG (CP Kelco) | Xanthan Gum | thickener. | 0.4000 | 100.00 | 0.4 |
| Dissolvine 220 | Tetrasodium EDTA | sequestering agent | 0.0500 | 100 | 0.05 |

| **PART C** | | | | | |
|---|---|---|---|---|---|
| Glycerine 99.5% | Glycerin | humectant | 2.0000 | 100 | 2 |
| Methylparaben | Methylparaben | preservative | 0.2000 | 100 | 0.2 |
| Propylparaben | Propylparaben | preservative | 0.0500 | 100 | 0.05 |

| **PART D** | | | | | |
|---|---|---|---|---|---|
| Liquid DL-Panthenol 50% | Panthenol | Moisturizer/anti-inflammatory | 0.5000 | 50 | 0.25 |
| Activera 1-200C | Aloe Barbadensis Leaf Juice | soothing/moisturizing agent | 0.0100 | 100 | 0.01 |
| Allantoin | Allantoin | anti-irritant/moisturizer | 0.3000 | 100 | 0.3 |
| Crodafos CES | Cetearyl Alcohol | emulsifier | 1.5000 | 65-75 | 1.05 |
| | Dicetyl Phosphate | | | 10-20 | 0.225 |
| | Ceteth-10 Phosphate | | | 10-20 | 0.225 |
| Montanov 68 | Cetearyl Alcohol | emulsifier | 1.5000 | 80 | 1.2 |
| | Cetearyl Glucoside | | | 20 | 0.3 |
| DOWANOL EPH | Phenoxyethanol | preservative | 0.7500 | 100 | 0.75 |
| Vitamin E Acetate | Tocopherol Acetate | antioxidant | 0.1000 | 100 | 0.1 |

| **PART E** | | | | | |
|---|---|---|---|---|---|
| Hetester PHA | Propylene Glycol Isoceteth-3 Acetate | emulsifier | 2.0000 | 100 | 2 |
| Lidocaine Base USP | Lidocaine | analgesic | 2.0000 | 100 | 2 |
| Dow Corning 245 | Cyclopentasiloxane | emollient | 2.0000 | 0 | 0 |

| **PART F** | | | | | |
|---|---|---|---|---|---|
| SYN. A BISABOLOL | Alfa-bisabolol | anti-inflammatory | 0.1000 | 100 | 0.1 |
| Hydrosoy 2000 | Hydrolized Soy Protein | conditioning agent | 0.2500 | 20 | 0.05 |
| Dry Flo PC | Aluminum Starch Octenylsuccinate | absorbent/feel modifier. | 10.0000 | 100 | 10 |
| Tapioca Pure | Tapioca Starch | absorbent/feel modifier. | 8.0000 | 100 | 8 |
| Boron Nitride CC 6064 Boron Nitride | | absorbent/feel modifier. | 2.0000 | 100 | 2 |
| MSS-500W | Silica | absorbent/feel modifier. | 2.0000 | 100 | 2 |
| KSP-100 | Vinyl Dimethicone/Methicone Silsesquioxane crosspolymer | absorbent/feel modifier. | 1.0000 | 100 | 1 |

| **PART G** | | | | | |
|---|---|---|---|---|---|
| Deionized Water | Water (Aqua) | | 5.0000 | 0 | 0 |
| OriStarDPG | Dipotassium Glycyrrhizate | anti-inflammatory | 0.1000 | 100 | 0.1 |
| | | | | | |

| | | | | | Total Solids |
|---|---|---|---|---|---|
| | | | 100 | | 34.36 |

### PROCEDURE:

PART A: Deionized water was metered into a main processing tank and high speed mixing started.
PART B: The ingredients of Part B were premixed and then added to Part A. The mixture was mixed until completely dispersed and then heated to 75°C. Mixing continued until the mixture was smooth and uniform.
PART C: At 75°C Part C ingredients were added. The mixture was mixed until all the solids were dissolved and the batch was uniform.
PART D: While maintaining the batch temperature at 75°C, Part D ingredients were added. The mixture was mixed until uniform and then cooled to 55°C.
PART E: At 55°C, premixed Part E ingredients were added and the mixture was mixed until uniform and then cooled to 40°C.
PART F: At 40°C Part F ingredient were added and the mixture was mixed until smooth and lump-free.
PART G: Premixed Part G ingredients were added to the main mix. Mixing continued until cooled to 35°C.
The final composition had a pH of 7.62 and a viscosity of 6500cps when measured with spindle #4 at 10rpm.

### EXAMPLE 2

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. The claims are meant to cover the claimed components and steps in any sequence which is effective to meet the objectives there intended, unless the context specifically indicates the contrary.

## Claims

1. A method of preparing a body surface for epilation, said method comprising prior to said epilation, applying to said body surface a composition that is adapted to leave a powder residue on said body surface, wherein said composition comprises a powder residue generating material and a fluid vehicle.

2. A method of claim 1, wherein said powder residue-generating material is selected from a group consisting of a water-insoluble solid, a water-soluble salt, and combinations thereof.

3. A method of claim 1, wherein said powder residue-generating material comprises a water-insoluble solid.

4. A method of claim 3, wherein said water-insoluble solid n comprises a material selected from a group consisting of an inorganic particulate, an organic particulate, a silicone particulate and combinations thereof.

5. A method of claim 1, wherein said powder residue-generating material comprises a water-soluble salt.

6. A method of claim 5, wherein said water-soluble salt comprises a material selected from a group consisting of an ionic surfactant, an ionic polymer, an alkali metal salt, an alkaline earth salt, and combinations thereof.

7. A method of claim 1, wherein said composition comprises a weight fraction of at least about 1% by weight of said powder residue generating material, wherein said weight fraction is relative to the composition.

8. A method of claim 1, wherein said composition comprises a weight fraction from about 3% by weight to about 60% by weight of a powder residue generating material, wherein said weight fraction is relative to the composition.

9. A method of claim 1, wherein said composition comprises a weight solids fraction of at least about 15% by weight of a powder residue generating material, wherein said weight solids fraction is relative to a total solids concentration in the composition.

10. A method of claim 1, wherein said composition comprises a weight solids fraction of from about 30% by weight to about 99% of a powder residue generating material, wherein said weight solids fraction is relative to a total solids concentration in the composition.

11. A method of claim 1, wherein said fluid vehicle comprises a water phase and an oil phase.

12. A method of claim 1, wherein said fluid vehicle comprises an oil phase emulsified in a water phase.

13. A method of claim 1, wherein said composition comprises a water insoluble solid, and wherein said fluid vehicle comprises an oil phase emulsified in a water phase.

14. A method of claim 1, wherein said composition comprises an analgesic.

15. A method of claim 14, wherein said analgesic is selected from benzocaine, dibucaine, lidocaine, benzyl alcohol, camphor, juniper tar, menthol, methyl nicotinate, methyl salicylate, phenol, resorcinol and mixtures thereof.

16. A method of claim 15, wherein said analgesic is lidocaine.

17. A method of claim 16, wherein said lidocaine is in basic form.

18. A method of claim 1, wherein said epilation comprises pulling said hair with sufficient force to extract said hair from said body surface.

19. A method of claim 1, wherein said composition is essentially free of chemical hair-removers.

20. A method of claim 1, wherein said composition is an oil in water emulsion, and wherein said composition comprises a powder residue generating material, wherein said powder residue generating material is present in a weight fraction from about 3% by weight to about 60% by weight of said composition, wherein said weight fraction is relative to the composition.
